# EUROPEAN PATENT SPECIFICATION

(11) **EP 1 567 125 B1**
(45) Date of publication and mention of the grant of the patent: **14.01.2009**
(21) Application number: 03809756.4
(22) Date of filing: 31.10.2003
(51) Int. Cl.: A61K 9/00, A61K 9/107

(54) **PHARMACEUTICAL COMPOSITIONS SUITABLE FOR THE TREATMENT OF OPHTHALMIC DISEASES**
PHARMAZEUTISCHE ZUSAMMENSETZUNGEN ZURBEHANDLUNG VON AUGENERKRANKUNGEN
COMPOSITIONS PHARMACEUTIQUES ADAPTEES AU TRAITEMENT DE MALADIES OPHTALMIQUES

(30) Priority: 31.10.2002 IT MI20022323
(43) Date of publication of application: 31.08.2005
(73) Proprietor: Gasco, Maria Rosa, I-10153 Torino (IT); Zara, Gian Paolo, I-10100 Torino (IT)
(72) Inventor: GASCO, Maria, Rosa, I-10153 Torino (IT); ZARA, Gian, Paolo, I-10100 Torino (IT); SAETTONE, Marco, Fabrizio, I-55049 Viareggio (IT)
(74) Representative: Gervasi, Gemma
(86) International application number: PCT/EP2003/012180
(87) International publication number: WO 2004/039351

(56) References cited:
- EP-A- 0 437 368
- WO-A-99/39700
- US-A- 5 662 932
- US-B1- 6 419 949
- GASCO M. R.: "Solid lipid nanospheres form warm micro-emulsions" PHARMACEUTICAL TECHNOLOGY EUROPE, vol. 9, no. 11, 1997, pages 52-58, XP009030038
- CAVALLI ROBERTA ET AL: "Solid lipid nanoparticles (SLN) as ocular delivery system for tobramycin" INTERNATIONAL JOURNAL OF PHARMACEUTICS (KIDLINGTON), vol. 238, no. 1-2, 15 May 2002 (2002-05-15), pages 241-245, XP002278435 ISSN: 0378-5173
- KOMATSU A ET AL: "Application of lipid microsphere drug delivery system to steroidal ophthalmic preparation." JAPANESE JOURNAL OF OPHTHALMOLOGY. JAPAN 1988, vol. 32, no. 1, 1988, pages 41-43, XP009029506 ISSN: 0021-5155

## Description

### Known art

Historically, the therapeutic treatment of the eye has been essentially directed towards the administration of drugs directly to the tissues and the fluids of the anterior segment of the eye.

Only recently has research been directed towards the administration of drugs intended for the posterior segment of the eye (uveal region, vitreous fluid, choroid and retina).

The eye is an isolated and highly protected organ.

In particular, the tight junctional complexes of the retinal pigmented epithelium and the retinal capillaries constitute the blood-retinal barrier for which the systemic administration of drugs does not succeed in reaching an adequate level within the posterior segment of the eye.

On the other hand, even with topical administration, only small amounts of drug reach the retina, as penetration through the external walls of the eye is very low. Nevertheless, there are numerous pathologies of the posterior segment of the eye which require pharmacological treatment such as for example bacterial or fungal endophthalmitis, viral retinitis, vitreoretinopathy, toxoplasmosis, uveitis, tumours, vascular diseases, diabetic retinopathy, age-related macular degeneration, glaucoma and others.

In order to overcome such difficulties, various methods of administration have been investigated.

Some authors have intravenously injected thermosensitive liposomes which have been lysed within the retinal vessels using microwave generated impulses (Khoobehi B. et al. Ophthalmology 1988 Jul, 95 (7): 950-5).

The injection of drugs into the vitreous fluid has also been described (Martidis A. et al. Ophthalmology 2002, 195 (5): 920-7).

However, frequently, this injection must be repeated and furthermore, can hold dangerous complications for the structures of the eye.

Finally, slow-release ocular implants have been proposed, such as for example Vitrasert^{®}, an implant which is surgically inserted into the eye and releases ganciclovir over a period of six months. (Morley MG. et al Ophthalmologhy 1996; 103 (10): 1517).

US-A-5 662 932 describes in general lipid emulsosomes for therapeutic applications. EP-A-437 368 describes lipid microparticles accessing the aqueous humour of the eye, cornea, iris and ciliary body. Pharm. Techn., Eur., 9(11),1997, p.52-58 refers of myotic effect of topically administered SLNs. Int. J. Pharm., 238, 2002 , p.241-245 describes the pre-ocular retention of nanoparticles in the aqueous humour and the corneal surface. Jpn. J. Ophthalm., 32, 1988, p.41-43 shows the permeation of the anterior part of the eye after topical administration of lipidic drug-loaded microspheres. US-A-6 419 949 describes generic compositions for oral or intravenous administration. WO-A- 99/39700 describes in general lipid nanoparticles, e.g. for oral use.

From that reported above, it is clearly evident that only through very complicated, time consuming and expensive methods, is it possible to convey drugs to the posterior segment of the eye.

### Summary

Now, pharmaceutical compositions suitable for the treatment of ophthalmic diseases have been found that allow to overcome the difficulties of the known art. Said compositions comprise solid lipidic nanoparticles (SLNs) having mean diameter comprised between 50 and 400 nm and preferably comprised between 100 and 200 nm wherein, within said nanoparticles, a pharmacologically active substance for the specific ophthalmic treatment is incorporated. Said compositions are prepared both in a form suitable for intravenous administration and a form suitable for topical ocular applications. It has been found that the solid lipidic nanoparticles of the present invention are able to transport the drug to the vitreous fluid and to the retina, through the above mentioned administration routes, overcoming the difficulties of the known art

### Detailed description of the invention

The present invention refers to the use of solid lipidic nanoparticles (SLNs) for the preparation of pharmaceutical compositions suitable for the treatment of ophthalmic diseases of the posterior segment of the eye. A pharmacologically active substance for the specific ophthalmic treatment is incorporated within said nanoparticles.

The nanoparticles containing the pharmacologically active substance are prepared essentially according to the process described in European patent N° 0526666 which comprises the following steps:
a) a molten lipid substance containing a drug or a complex thereof is mixed with a mixture comprising and preferably consisting of water, a surfactant, a cosurfactant and optionally a counterion of the drug, pre-warmed to a temperature at least equal to the melting temperature of said lipid substance, thus obtaining a microemulsion having a temperature at least equal to the melting temperature of said lipid substance;
b) the microemulsion obtained in step a) is dispersed in water or in an aqueous medium cooled to a temperature comprised of between 2 and 5 °C, thus obtaining a dispersion of solid lipidic nanoparticles incorporating the drug;
c) the dispersion obtained in step b) is washed with water or with an aqueous medium by diafiltration with the practically total elimination of the surfactant and cosurfactant;
d) the dispersion obtained as in step c) is dried by lyophilisation or by spray drying or by evaporation, thus obtaining the solid lipidic nanoparticles (SLNs) with the incorporated drug.

The microemulsion of step a) can be sterilised by filtration using sterilising filters. The dispersion obtained in step c) can be sterilised in an autoclave or by filtration using sterilising filters.

According to an alternative embodiment, the microemulsion obtained in step a) is added to a mixture comprising, and preferably consisting of, water, a surfactant, a cosurfactant and a lipid, warmed to a temperature at least equal to the melting temperature of the lipid and the mixture thus obtained is dispersed in water or in an aqueous medium cooled to a temperature comprised of between 2 and 5°C. According to an additional alternative embodiment, at the end of step a) a substance suitable to sterically stabilise the lipidic nanoparticles is added. The lipidic substances used in the process are selected from the group comprising:
- triglycerides, particularly trilaurine, tricapriloin, tristearine, tripalmitine, capric/caprylic triglycerides (Mygliol^{®}, Captex^{®} and Labrafac^{®});
- diglycerides, particularly dipalmitine and distearine;
- monoglycerides, particularly glyceryl monostearate (Myvapex 600^{®}) and glyceryl palmitostearate (Precirol^{®});
- aliphatic alcohols, particularly cetylic alcohol and stearylic alcohol;
- fatty acids having C10 - C22 chains, decanoic acid, linoleic acid and polyalcohol esters thereof;
- cholesterol and esters thereof, particularly cholesteryl hemisuccinate, cholesteryl butyrate and cholesteryl palmitate.

The surfactants are selected from the group comprising:
- lecithins, as they are, such as Lipoid 75^{®} and Epicuron 200^{®}, phospholipids and hydrogenated forms thereof and synthetic and semi-synthetic derivatives thereof;
- bile salts, particularly sodium glycocholate, sodium taurocholate and taurodeoxycholate;
- Tween 20, Tween 40, Tween 80, Span 20, Span 40 and Span 60;
- emulsifiers, particularly gelatin.

The cosurfactants are selected from the group comprising:
- low molecular weight alcohols or glycols, particularly butanol, hexanol and hexadiol;
- low molecular weight fatty acids, particularly butyric acid and octanoic acid;
- phosphoric acid esters, benzylic alcohol and bile salts. The substances suitable to sterically stabilise the lipidic nanoparticles are selected from dipalmitoyl phosphatidyl ethandiamine-PEG, diacyl phosphatidyl ethanolamine PEG (PEG M.W. 750-2000) and fatty acids pegylated with PEG-methylethers (PEG M.W. 750-2000).

The pharmacologically active substances suitable for the treatment of ophthalmic diseases according to the present invention can be both of the hydrophilic type and of the hydrophobic type and comprise antibiotics, antifungal agents, antiviral agents, antineoplastics, drugs for diabetic retinopathy, steroidal and non-steroidal anti-inflammatory agents, and antiglaucoma drugs. Preferably said pharmacologically active substances are selected from the group comprising: amphotericin, miconazole, ganciclovir, saquinavir, acyclovir, famciclovir, vidarabine, idoxuridine, β-interferon, paclitaxel, methotrexate, doxorubicin, angiopoietin 1, diclophenac, indomethacin, ketorolac, piroxicam, flurbiprofen, dexamethasone, triamcinolone, hydrocortisone, fluorometholone, rimexolone, timolol, betaxolol and acetazolamide.

The solid lipidic nanoparticles (SLNs) of the present invention have a mean diameter comprised between 50 and 400 nm and preferably comprised between 100 and 200 nm and a polydispersion comprised between 0.06 and 0.30 and preferably comprised between 0.10 and 0.20.

Said SLNs have a pharmacologically active substance content comprised between 0.1 and 7.0 %.

They are used for the preparation of pharmaceutical compositions for intravenous administration or for topical ocular administration.

The compositions for intravenous administration are prepared by dispersion of the SLNs in isotonic aqueous solutions in such quantities as to obtain a concentration of SLNs comprised between 10 and 250 mg/ml.

Preferably said aqueous solution is made isotonic by the addition of glycerol.

The compositions for topical ocular administration are prepared in the same manner with the further addition of 0.1-0.4% of a viscosizing substance, for example polyvinyl alcohol or hydroxypropyl cellulose, and contain 1.0 to 25% w/v SLNs.

A therapeutic method for the treatment of ophthalmic diseases comprises, and preferably consists in, the intravenous or topical ocular administration of a therapeutically effective amount of a pharmaceutical composition as defined above.

The dosage for intravenous administration is of an amount of composition containing 0.01-5.0 milligrams of active substance per kilogram of body weight. The dosage for topical ocular administration is of an amount of composition containing 0.01-5.0 mg of active substance per eye.

The compositions according to the present invention have important advantages compared to the known art with regard to both the simplicity of preparation and application and the efficacy of the active substance.

Indeed they allow the transport of the SLNs to the posterior segment of the eye hollowing both systemic and topical ocular administration.

In any case, the blood-retinal barrier is easily overcome and the active substance incorporated within the SLNs reaches the vitreous fluid and the retina. It shall be noted that said compositions allow the transport across the blood-retinal barrier even of active substances that are practically insoluble in an aqueous medium.

Finally, the compositions for intravenous administration can be constituted by sterically stabilised SLNs as already observed, with the advantage of minimising their uptake by macrophages.

For the purpose of illustration of the preparation process of the solid lipidic nanoparticles, of the product obtained and of the effects of its ophthalmic administration, the following examples are reported.

### Example 1 (Preparation of the SLNs)

200 mg of molten stearic acid at a temperature of 70°C containing a 1:2 gentamicin-hexadecylphosphate (28.85 mg, equivalent to 12 mg of gentamicin) complex are added to a mixture constituted by filtered water (2 ml), Epikuron 200^{®} (105 mg) and sodium taurocholate (285 mg) warmed to a temperature of 70 °C.

The microemulsion obtained, having a temperature of 70 °C, is dispersed in water in a volume ratio of 1/5 at a temperature of 2-3°C by mechanical stirring obtaining a dispersion of solid lipidic nanoparticles (SLNs). The dispersion obtained is washed twice with water for injection by diafiltration.

The SLNs have a mean diameter of 75 nm and a polydispersion of 0.2 and the lyophilised product has a gentamicin content of 3.3%.

### Example 2 (intravenous administration)

An isotonic aqueous dispersion has been prepared with the solid lipidic nanoparticles (SLNs) prepared according to example 1, having a concentration of SLNs corresponding to 6 mg/ml of gentamicin.

The dispersion has been injected into the marginal ear vein of three male New Zealand albino rabbits having weights of 2.8-3.5 kg. The injected dose of gentamicin has been 1.5 mg/kg.

The commercial composition Gentomil^{®}, containing the same dose of gentamicin, has been injected as a control into other three rabbits having the same characteristics.

One hour after administration, the following results, which represent the mean values of the gentamicin concentrations in various ocular areas, have been obtained.
(a) Dispersion of SLNs:
   - concentration of gentamicin in the aqueous fluid:
      right eye = 300 ng/100 µl
      left eye = 326 ng/100 µl
   - concentration of gentamicin in the vitreous fluid:
      right eye = 499 ng/100 µl
      left eye = 531 ng/100 µl
   - concentration of gentamicin in the retina:
      right eye = 1225 ng/100 µl
      left eye =1365 ng/100 µl
(b) Gentomil^{®} composition
   - Concentration of gentamicin in the aqueous fluid:
      right eye = 50 ng/100 µl
      left eye = 56 ng/100 µl
   - Concentration of gentamicin in the vitreous fluid:
      right eye = 3,5 ng/100 µl
      left eye = 2,5 ng/100 µl
   - Concentration of gentamicin in the retina: non perceptible.

### Example 3 (intravenous administration)

Example 2 has been repeated with the difference that the dose injected has been 2 mg/kg.

Three hours after administration, the following results have been obtained.
(a) Dispersion of SLNs:
   - concentration of gentamicin in the aqueous fluid:
      right eye = 244 ng/100 µl
      left eye = 120 ng/100 µl
   - concentration of gentamicin in the vitreous fluid:
      right eye = 126 ng/100 µl
      left eye = 157 ng/100 µl
   - concentration of gentamicin in the retina:
      right eye = 99,5 ng/100 µl
      left eye = 84 mg/100 µl
(b) Gentomil^{®} composition
   - Concentration of gentamicin in the aqueous fluid:
      right eye = 40 ng/100 µl
      left eye = 36 ng/100 µl
   - Concentration of gentamicin in the vitreous fluid:
      not perceptible
   - Concentration of gentamicin in the retina:
      not perceptible

### Example 4 (topical ocular administration)

An isotonic aqueous dispersion has been prepared with the solid lipid nanoparticles (SLNs) prepared according to example 1, having a concentration of SLNs corresponding to 2 mg/ml of gentamicin.

Polyvinyl alcohol (M.W. 20,000) has been added to the dispersion as a viscosizing agent, in an amount of 0.2% with respect to the dispersion.

Three rabbits having the characteristics described in example 2 have been used for the experiment.

The administration has been carried out by topically administering 50 µl of SLNs dispersion into the lower conjunctival sack of one eye of each rabbit. As a control, the same dose of gentamicin has been administered in the same manner to other three rabbits having the same characteristics, by means of a commercial composition denominated Genticol^{®}.

One hour after administration, the following results, which represent the mean values of the concentrations of gentamicin within the eye, have been obtained.
(a) Dispersion of SLNs:
   - Concentration of gentamicin in the aqueous fluid = 10 µg/100 µl
   - concentration of gentamicin in the vitreous fluid = 2.76 µg/100 µl
   - concentration of gentamicin in the retina = 890 ng/100 µl
(b) Genticol^{®} composition
   - Concentration of gentamicin in the aqueous fluid = 5 µg/100 µl
   - Concentration of gentamicin in the vitreous fluid: not perceptible
   - Concentration of gentamicin in the retina: not perceptible.

### Example 5 (topical administration)

Example 4 has been repeated with the difference that a dose of 200 µl has been administered.

One hour after administration the following results have been obtained which represent the mean values of the concentrations of gentamicin in the eye.
(a) Dispersion of SLNs:
   - concentration of gentamicin in the aqueous fluid = 35µg/100µl
   - concentration of gentamicin in the vitreous fluid = 7.84µg/100µl
   - concentration of gentamicin in the retina = 5.4µg/100µl
(b) Genticol^{®} composition
   - concentration of gentamicin in the aqueous fluid = 16µg/100µl
   - concentration of gentamicin in the vitreous fluid = trace
   - concentration of gentamicin in the retina = trace.

## Claims

1. Use of solid lipidic nanoparticles (SLNs) for the preparation pharmaceutical compositions for the treatment of ophthalmic diseases of the posterior segment of the eye by intravenous or topical ocular administration, wherein in said SLNs is incorporated a pharmacologically active substance for the treatment of said diseases.

2. The use according to claim 1, **characterised in that** said SLNs have a mean diameter comprised between 50 and 400 nm, and a polydispersion comprised of between 0.06 and 0.30.

3. The use according to claim 1, **characterised in that** said SLNs have an average diameter comprised between 100 and 200 nm and a polydispersion comprised of between 0.10 and 0.20.

4. The use according to claim 1, **characterised in that** said SLNs have a pharmacologically active substance content comprised of between 0.1 and 7.0%.

5. The use according to claim 1, **characterised in that** said pharmaceutical compositions for the treatment of said ophthalmic diseases by intravenous. administration consist essentially of dispersions of said SLNs in isotonic aqueous solutions having a concentration of SLNs comprised between 10 and 250 mg/ml.

6. The use according to claim 1, **characterised in that** said pharmaceutical compositions for the treatment of said ophthalmic diseases by topical ocular administration consist essentially of dispersions of said SLNs in isotonic aqueous solution having a concentration of SLNs comprised between 1.0 and 25% w/v and further containing from 0.1 to 0.4 % of a viscosizing substance.

7. The use according to claim 1, **characterised in that** said pharmacologically active substance is selected from the group comprising: amphotericin, miconazole, ganciclovir, saquinavir, acyclovir, famciclovir, vidarabine, idoxuridine, β-interferon, paclitaxel, methotrexate, doxorubicin, angiopoietin 1, diclophenac, indomethacin, ketorolac, piroxicam, flurbiprofen, dexamethasone, triamcinolone, hydrocortisone, fluorometholone, rimexolone, timolol, betaxolol and acetazolamide.

8. The use according to claim 1, **characterised in that** said SLNs are prepared by a process wherein:
a) a molten lipid substance containing a drug or its complex is mixed with a mixture comprising water, a surfactant, a cosurfactant and optionally a counterion of the drug, pre-warmed to a temperature at least equal to the melting temperature of said lipid substance, thus obtaining a microemulsion having a temperature at least equal to the melting temperature of said lipid substance;
b) the microemulsion obtained in step a) is dispersed in water or in an aqueous medium cooled to a temperature comprised between 2 and 5 °C, thus obtaining a dispersion of solid lipidic nanoparticles incorporating the drug;
c) the dispersion obtained in step b) is washed with water or with an aqueous medium by diafiltration with the practically total elimination of the surfactant and the cosurfactant;
d) the dispersion obtained in step c) is dried by lyophilisation or by spray drying or by evaporation, thus obtaining the solid lipid nanoparticles (SLNs) with the drug incorporated.

9. The use according to claim 8; **characterised in that** the microemulsion obtained in step a) is added to a mixture comprising water, a surfactant, a cosurfactant and a lipid warmed to a temperature at least equal to the melting temperature of the lipid and the mixture thus obtained is dispersed in water or in an aqueous medium cooled to a temperature comprised of between 2 and 5°C.

10. The use according to claim 8, **characterised in that** at the end of step a) a substance suitable for stabilising the SLNs is added selected from the group comprising dipalmitoyl phosphatidyl ethanolamine-PEG, diacyl phosphatidyl ethanolamine-PEG (PEG M. W. 750-2000) and fatty acids pegylated with PEG-methylethers (PEG M. W. 750-2000).

11. A pharmaceutic composition for the treatment of ophthalmic diseases of the posterior segment of the eye by intravenous or topical ocular administration, consisting essentially of an isotonic aqueous dispersion of solid lipid nanoparticles (SLNs) having a mean diameter comprised between 50 and 400 nm and polydispersion comprised between 0.06 and 0.30, a pharmacologically active substance for the treatment of said diseases being incorporated within said SLNs.

12. The pharmaceutical composition according to claim 11, **characterised in that** said aqueous dispersion contains a viscosizing substance.

13. The composition according to claim 11, **characterised in that** said SLNs have a mean diameter comprised between 100 and 200 nm and polydispersion comprised between 0.10 and 0.20.

14. The composition according to claim 11, **characterised in that** for the intravenous administration, said isotonic aqueous dispersion has a concentration of SLNs comprised of between 10 and 250 mg/ml.

15. The composition according to claim 11, **characterised in that** for the topical ocular administration, said isotonic aqueous dispersion has a concentration of SLNs comprised between 1 and 25% w/v and contains from 0.1 to 0.4% of a viscosizing substance.

16. The composition according to claim 11, **characterised in that** said SLNs have a pharmacologically active substance content comprised between 0.1 and 7.0%:

17. The composition according to claim 11, **characterised in that** said pharmacologically active substance is selected from the group comprising: amphotericin, miconazole, ganciclovir, saquinavir, acyclovir, famciclovir, vidarabine, idoxuridine, ß-interferon, paclitaxel, methotrexate, doxorubicin, angiopoietin 1, diclophenac, indomethacin, ketorolac, piroxicam, flurbiprofen, dexamethasone, triamcinolone, hydrocortisone, fluorometholone, rimexolone, timolol, betaxolol e acetazolamide.

18. Compositions according to claim 11, **characterised in that** the lipid of said SLNs is selected from the group comprising trilaurine, tricapriloin, tristearine, tripalmitine, capric/caprylic triglycerides, dipalmitine, distearine, glyceryl monostearate, glyceryl palmitostearate, cetylic alcohol, stearylic alcohol, fatty acids having C10-C22 chains, cholesteryl hemisuccinate, cholesteryl butyrate and cholesteryl palmitate.

## Patentansprüche

1. Verwendung von festen Lipid-Nanopartikeln (SLN'n) zur Herstellung von pharmazeutischen Zusammensetzungen für die Behandlung von Augenkrankheiten des Hinterabschnitts des Auges durch intravenöse oder topische Augenverabreichung, wobei in den SLN'n eine pharmakologisch aktive Verbindung für die Behandlung der Krankheiten inkorporiert ist.

2. Verwendung nach Anspruch 1, **dadurch gekennzeichnet, dass** die SLN einen durchschnittlichen Durchmesser zwischen 50 und 400 nm sowie eine Polydispersion zwischen 0,06 und 0,30 aufweisen.

3. Verwendung nach Anspruch 1, **dadurch gekennzeichnet, dass** die SLN einen durchschnittlichen Durchmesser zwischen 100 und 200 nm sowie eine Polydispersion zwischen 0,10 und 0,20 aufweisen.

4. Verwendung nach Anspruch 1, **dadurch gekennzeichnet, dass** die SLN einen Gehalt an pharmakologisch aktiver Verbindung zwischen 0,1 und 7,0 % aufweisen.

5. Verwendung nach Anspruch 1, **dadurch gekennzeichnet, dass** die pharmazeutischen Zusammensetzungen für die Behandlung der Augenkrankheiten durch intravenöse Verabreichung im Wesentlichen aus Dispersionen der SLN in isotonischen wässrigen Lösungen mit einer Konzentration der SLN zwischen 10 und 250 mg/ml bestehen.

6. Verwendung nach Anspruch 1, **dadurch gekennzeichnet, dass** die pharmazeutischen Zusammensetzungen für die Behandlung der Augenkrankheiten durch topische Augenverarbreichung im Wesentlichen aus Dispersionen der SLN in isotonisch wässriger Lösung mit einer Konzentration der SLN zwischen 1,0 und 25 % (Gew./Vol.) bestehen und des Weiteren zwischen 0,1 und 0,4 % eines Viskositätsmittels enthalten.

7. Verwendung nach Anspruch 1, **dadurch gekennzeichnet, dass** die pharmakologisch aktive Verbindung aus der Gruppe ausgewählt wird, welche besteht aus: Amphotericin, Miconazol, Ganciclovir, Saquinavir, Aciclovir, Famciclovir, Vidarabin, Idoxuridin, β-Interferon, Paclitaxel, Methotrexat, Doxorubicin, Angiopoietin 1, Diclophenac, Indomethacin, Ketorolac, Piroxicam, Flurbiprofen, Dexamethason, Triamcinolon, Hydrocortison, Fluormetholon, Rimexolon, Timolol, Betaxolol und Acetazolamid.

8. Verwendung nach Anspruch 1, **dadurch gekennzeichnet, dass** die SLN durch ein Verfahren hergestellt werden, bei dem:
a) eine geschmolzene, ein Arzneimittel oder dessen Komplex enthaltende Lipidverbindung mit einer Mischung vermischt wird, welche Wasser, ein Tensid, ein Cotensid und optional ein Gegenion des Arzneimittels enthält, wobei die Mischung auf eine Temperatur von wenigstens gleich der Schmelztemperatur der Lipidverbindung vorgewärmt ist, wodurch eine Mikroemulsion mit einer Temperatur von wenigstens gleich der Schmelztemperatur der Lipidverbindung erhalten wird,
b) die in dem Schritt a) erhaltene Mikroemulsion in Wasser oder in einem wässrigen Medium, welches auf eine Temperatur zwischen 2 und 5 °C abgekühlt ist, dispergiert wird, wodurch eine Dispersion von festen Lipidnanopartikeln, welche das Arzneimittel inkorporiert enthalten, erhalten wird,
c) die in dem Schritt b) erhaltene Dispersion mit Wasser oder mit einem wässrigen Medium durch Diafiltration unter praktisch vollständiger Entfernung des Tensids und des Cotensids gewaschen wird,
d) die in dem Schritt c) erhaltene Dispersion durch Lyophilisation oder durch Sprühtrocknen oder durch Verdampfung getrocknet wird, wodurch die festen Lipidnanopartikel (SLN) mit dem darin inkorporierten Arzneimittel erhalten werden.

9. Verwendung nach Anspruch 8, **dadurch gekennzeichnet, dass** die in dem Schritt a) erhaltene Mikroemulsion zu einer Mischung, welche Wasser, ein Tensid, ein Cotensid und ein Lipid enthält, zugegeben wird, wobei die Mischung auf eine Temperatur von wenigstens gleich der Schmelztemperatur des Lipids erwärmt ist, und, dass die so erhaltene Mischung in Wasser oder in einem wässrigen Medium, welches auf eine Temperatur zwischen 2 und 5 °C abgekühlt ist, dispergiert wird.

10. Verwendung nach Anspruch 8, **dadurch gekennzeichnet, dass** an dem Ende des Schritts a) eine zur Stabilisierung der SLN geeignete Verbindung zugegeben wird, welche aus der Gruppe ausgewählt ist, welche Dipalmitoylphosphatidylethanolamin-PEG, Diacylphosphatidylethanolamin-PEG (PEG mit einem MW zwischen 750 und 2000) und Fettsäuren pegyliert mit PEG-Methylethern (PEG mit einem MW zwischen 750 und 2000) enthält.

11. Pharmazeutische Zusammensetzung für die Behandlung von Augenkrankheiten des Hinterabschnitts des Auges durch intravenöse oder topische Augenverabreichung bestehend im Wesentlichen aus einer isotonischen wässrigen Dispersion von festen Lipidnanopartikeln (SLN'n) mit einem durchschnittlichen Durchmesser zwischen 50 und 400 nm und mit einer Polydispersion zwischen 0,06 und 0,30, wobei in den SLN'n eine pharamakologisch aktive Verbindung zur Behandlung der Krankheiten inkorporiert ist.

12. Pharmazeutische Zusammensetzung nach Anspruch 11, **dadurch gekennzeichnet, dass** die wässrige Dispersion ein Viskositätsmittel enthält.

13. Zusammensetzung nach Anspruch 11, **dadurch gekennzeichnet, dass** die SLN einen durchschnittlichen Durchmesser zwischen 100 und 200 nm sowie eine Polydispersion zwischen 0,10 und 0,20 aufweisen.

14. Zusammensetzung nach Anspruch 11, **dadurch gekennzeichnet, dass** die isotonische wässrige Dispersion für die intravenöse Verabreichung eine Konzentration der SLN zwischen 10 und 250 mg/ml aufweist.

15. Zusammensetzung nach Anspruch 11, **dadurch gekennzeichnet, dass** die isotonische wässrige Dispersion für die topische Augenverabreichung eine Konzentration der SLN zwischen 1 und 25 % (Gew./Vol.) aufweist und 0,1 bis 0,4 % eines Viskositätsmittels enthält.

16. Zusammensetzung nach Anspruch 14, **dadurch gekennzeichnet, dass** die SLN eine pharmakologisch aktive Verbindung in einer Menge zwischen 0,1 und 7,0 % aufweisen.

17. Zusammensetzung nach Anspruch 11, **dadurch gekennzeichnet, dass** die pharmakologisch aktive Verbindung aus der Gruppe ausgewählt ist, welche besteht aus: Amphotericin, Miconazol, Ganciclovir, Saquinavir, Aciclovir, Famciclovir, Vidarabin, Idoxuridin, β-Interferon, Paclitaxel, Methotrexat, Doxorubicin, Angiopoietin 1, Diclophenac, Indomethacin, Ketorolac, Piroxicam, Flurbiprofen, Dexamethason, Triamcinolon, Hydrocortison, Fluormetholon, Rimexolon, Timolol, Betaxolol und Acetazolamid.

18. Zusammensetzung nach Anspruch 11, **dadurch gekennzeichnet, dass** das Lipid der SLN aus der Gruppe ausgewählt ist, welche aus Trilaurin, Tricaprylin, Tristearin, Tripalmitin, Caprin-/Capryl-Triglyceriden, Dipalmitin, Distearin, Glycerylmonostearat, Glycerylpalmitostearat, Cetylalkohol, Stearylalkohol, Fettsäuren mit C₁₀-C₂₂-Ketten, Cholesterylhemisuccinat, Cholesterylbutyrat und Cholesterylpalmitat besteht.

## Revendications

1. Utilisation de nanoparticules lipidiques solides (SLN) pour la préparation de compositions pharmaceutiques pour le traitement des maladies ophtalmiques du segment postérieur de l'oeil par administration oculaire intraveineuse ou topique, où, dans lesdites SLN, est incorporée une substance pharmacologiquement active pour le traitement desdites maladies.

2. Utilisation selon la revendication 1, **caractérisée en ce que** lesdites SLN ont un diamètre moyen compris entre environ 50 et 400 nm, et une polydispersion comprise entre 0,06 et 0,30.

3. Utilisation selon la revendication 1, **caractérisée en ce que** lesdites SLN ont un diamètre moyen compris entre 100 et 200 nm et une polydispersion comprise entre 0,10 et 0,20.

4. Utilisation selon la revendication 1, **caractérisée en ce que** lesdits SLN ont une teneur en substances pharmacologiquement actives comprise entre 0,1 et 7,0%.

5. Utilisation selon la revendication 1, **caractérisée en ce que** lesdites compositions pharmaceutiques pour le traitement desdites maladies ophtalmiques par administration intraveineuse consistent essentiellement en dispersion desdites SLN dans des solutions aqueuses isotoniques ayant une concentration de SLN comprise entre 10 et 250 mg/ml.

6. Utilisation selon la revendication 1, **caractérisée en ce que** lesdites compositions pharmaceutiques pour le traitement desdites maladies ophtalmiques par administration oculaire topique consistent essentiellement en dispersion desdites SLN dans une solution aqueuse isotonique ayant une concentration de SLN comprise entre 1,0 et 25% p/v et contenant de plus de 0,1 à 0,4% d'une substance viscosifiante.

7. Utilisation selon la revendication 1, **caractérisée en ce que** ladite substance pharmacologiquement active est sélectionnée dans le groupe comprenant: amphotéricine, miconazole, ganciclovir, saquinavir, acyclovir, famciclovir, vidarabine, idoxuridine, β-interféron, paclitaxel, méthotrexate, doxorubicine, angiopoïétine 1, diclophénac, indométhacine, cétorolac, piroxicam, flurbiprofène, dexaméthasone, triamcinolone, hydrocortisone, fluorométholone, rimexolone, timolol, betaxolol et acétazolamide.

8. Utilisation selon la revendication 1, **caractérisée en ce que** lesdits SLN sont préparées par un procédé où:
a) une substance lipidique fondue contenant un médicament ou son complexe est mélangée à un mélange comprenant de l'eau, un agent tensioactif, un co-agent tensioactif et facultativement un contre-ion du médicament, préchauffé à une température au moins égale à la température de fusion de ladite substance lipidique, pour ainsi obtenir une microémulsion ayant une température au moins égale à la température de fusion de ladite substance lipidique;
b) la microémulsion obtenue à l'étape a) est dispersée dans l'eau ou dans un milieu aqueux refroidi à une température comprise entre 2 et 5°C, pour ainsi obtenir une dispersion de nanoparticules lipidiques solides incorporant le médicament;
c) la dispersion obtenue à l'étape b) est lavée avec de l'eau ou avec un milieu aqueux par diafiltration avec l'élimination pratiquement totale de l'agent tensioactif et du co-agent tensioactif;
d) la dispersion obtenue à l'étape c) est séchée par lyophilisation ou par séchage par pulvérisation ou par évaporation, obtenant ainsi les nanoparticules lipidiques solides (SLN) où est incorporé le médicament.

9. Utilisation selon la revendication 8, **caractérisée en ce que** la microémulsion obtenue à l'étape a) est ajoutée à un mélange comprenant de l'eau, un agent tensioactif, un co-agent tensioactif et un lipide chauffé à une température au moins égale à la température de fusion du lipide et le mélange ainsi obtenu est dispersé dans l'eau ou dans un milieu aqueux refroidi à une température comprise entre 2 et 5°C.

10. Utilisation selon la revendication 8, **caractérisée en ce qu'**à la fin de l'étape a), une substance appropriée pour stabiliser les SLN est ajoutée, sélectionnée dans le groupe consistant dipalmitoyl phosphatidyl éthanolamine-PEG, diacyl phosphatidyl éthanolamine-PEG (PEG P.M. 750-2000) et des acides gras pégylés avec des PEG-méthyléthers (PEG P.M. 750-2000).

11. Composition pharmaceutique pour le traitement de maladies ophtalmiques du segment postérieur de l'oeil par administration oculaire intraveineuse ou topique consistant essentiellement en une dispersion aqueuse isotonique de nanoparticules lipidiques solides (SLN) ayant un diamètre moyen compris entre 50 et 400 nm et une polydispersion comprise entre 0,06 et 0,30, une substance pharmacologiquement active pour le traitement des maladies étant incorporée dans lesdites SLN.

12. Composition pharmaceutique selon la revendication 11, **caractérisée en ce que** ladite dispersion aqueuse contient une substance rendant viscosifiante.

13. Composition selon la revendication 11, **caractérisée en ce que** lesdites SLN ont un diamètre moyen compris entre 100 et 200 nm et une polydispersion comprise entre 0,10 et 0,20.

14. Composition selon la revendication 11, **caractérisée en ce que** pour l'administration intraveineuse, ladite dispersion aqueuse isotonique a une concentration en SLN comprise entre 10 et 250 mg/ml.

15. Composition selon la revendication 11, **caractérisée en ce que** pour l'administration oculaire topique, ladite dispersion aqueuse isotonique a une concentration en SLN comprise entre 1 et 25% p/v et contient de 0,1 à 0,4% d'une substance viscosifiante.

16. Composition selon la revendication 11, **caractérisée en ce que** lesdites SLN ont une teneur en substance pharmacologiquement active comprise entre 0,1 et 7,0%.

17. Composition selon la revendication 11, **caractérisée en ce que** ladite substance pharmacologiquement active est sélectionnée dans le groupe consistant en: amphétoricine, miconazole, ganciclovir, saquinavir, acyclovir, famciclovir, vidarabine, idoxuridine, β-interféron, paclitaxel, méthorexate, doxorubicine, angiopoiétine 1, diclophénac, indométhacine, cétorolac, piroxicam, flurbiprofène, dexaméthasone, triamcinolone, hydrocortisone, fluorométholone, riméxolone, timolol, bétaxolol et acétazolamide.

18. Composition selon la revendication 11, **caractérisée en ce que** le lipide desdites SLN est sélectionnée dans le groupe comprenant trilaurine, tricapriloïne, tristéarine, tripalmitine, triglycérides caprique/caprylique, dipalmitine, distéarine, monostéarate de glycéryle, palmitostéarate de glycéryle, alcool cétylique, alcool stéarylique, acides gras ayant des chaînes C10-C22, hémisuccinate de cholestéryle, butyrate de cholestéryle et palmitate de cholestéryle.
